# EUROPEAN PATENT SPECIFICATION

(11) **EP 1 515 663 B1**
(45) Date of publication and mention of the grant of the patent: **26.10.2011**
(21) Application number: 03728871.9
(22) Date of filing: 13.05.2003
(51) Int. Cl.: A61F 2/00

(54) **STENT WITH POLAR RADIOPAQUE MARKER**
STENT MIT POLARER STRAHLUNGSUNDURCHLÄSSIGER MARKIERUNG
STENT AVEC MARQUEUR RADIO-OPAQUE POLAIRE

(30) Priority: 28.05.2002 US 63937
(43) Date of publication of application: 23.03.2005
(73) Proprietor: Boston Scientific Limited, St. Michael, Barbados, West Indies (BB)
(72) Inventor: GERBERDING, Brent, San Jose, CA 95126 (US)
(74) Representative: Hauck Patent- und Rechtsanwälte
(86) International application number: PCT/US2003/015051
(87) International publication number: WO 2003/101343

(56) References cited:
- EP-A2- 1 433 438
- EP-A2- 1 433 438
- WO-A-00/62710
- US-A1- 2001 027 339
- US-A1- 2002 027 339
- US-A1- 2002 027 339
- US-B1- 6 334 871

## Description

### Background of Invention

The use of stents in bodily lumen is well known. Stents have been used in a wide range of bodily vessels including coronary arteries, renal arteries, peripheral arteries including iliac arteries, arteries of the neck and cerebral arteries, biliary ducts, urethras, fallopian tubes, bronchial tubes, the trachea, the esophagus, the prostate and the bowels. The size and design of a stent may vary, depending on the location where the stent is needed.

A stent is typically delivered in an unexpanded state to a desired location in a bodily lumen and then expanded. The stent may be expanded via the use of mechanical device such as a balloon or the stent may be self-expanding.

A stents is typically delivered to a desired location In a bodily vessel via the use of a catheter. The stent, whether balloon-expandable, self-expanding or both, is disposed about a portion of the catheter and delivered via tortuous vessels to the target region of a vessel.

In order to monitor the delivery and placement of the stent, the stent typically will have a radiopaque region which may be viewed via fluoroscopy. In some cases, the radiopaque region is provided by differentially plating or coating a region of the stent. In other cases, rivets or inserts of radiopaque material are added to the stent. The use of rivets or other inserts may be of particular value where the individual struts of the stent are less than 0.0127cms (0.005 inches) wide. The presence of rivets or inserts, however, absent any other modifications to the stent may reduce the extent to which the stent may be crimped and the uniformity of such a crimp. Moreover, the struts adjacent to the rivets or inserts may be damaged by the rivets or inserts during crimping of the stent..

Stents may be used without coverings or with coverings. Covered stents typically have a covering disposed about the stent to prevent the flow of fluid through the sidewalls. The entirety of the stent may be covered or only a portion of the stent may be covered. Coverings on stents may advantageously be used in the region of aneurysms to prevent leakage of blood. Positioning the covered portion of the stent in the desired region in the vessel, however, may prove challenging.
WO 00/62710 describes a medical device including a catheter and a stent mounted on the catheter. The stent having a hollow, cylindrical body made with a plurality of rings. The rings each extends circumferentially around the cylindrical body and include an undulating series of peaks and valleys. The rings are joined together by a series of links which are shaped and arranged to promote longitudinal flexibility as the stent is delivered on the catheter and effective scaffolding after deployment. The rings are provided with inflection points on some portions of the rings which extend in generally circumferential direction for short distance. A link is joined at one end of the inflection point on one ring and also joined to a second end at a second inflection point on an adjacent ring, This construction allows the crimped stent to flex longitudinally when it is subjected to bending forces such as those encountered during delivery of the stent and catheter through a tortuous coronary artery.
There remains a need for a medical device with radiopaque markers which may be crimped to smaller sizes in general and for covered stents having radiopaque markers which may be crimped to smaller sizes. There also remains a need for stonts with specialized coverings to selectively cover desired portions of the stents.

### Summary of Invention

The present invention provides a medical device as defined in independent claim 1. Without limiting the scope of the invention, a brief summary of the claimed embodiments of the invention is set forth below. Additional details of the summarized embodiments of the invention and/or additional embodiments of the invention may be found in the detailed description of the invention below and the dependent claims.

The special strut is located between the ends of the medical device. In one embodiment, the special strut is located between the middle of the medical device and a location one third of the way along the medical device.

According to the invention, the medical device further comprises a cover disposed thereabout, at least in the vicinity of the special strut.

The medical device will typically be in the form of a stent but may also be provided in the form of a bifurcared stent, stent-graft, graft, filter, occlusive device.

Additional details and/or embodiments of the invention are discussed below.

### Brief Description of Drawings

Figs. 1a-1f show flat views of inventive stents.

Fig. 2a shows a schematic illustration of an inventive stent including a covered portion.

Figs. 2b-2e show other embodiments of inventive stents with coverings.

### Detailed Description

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated. Also for the purposes of this disclosure, the term stent includes within its scope stent grafts.

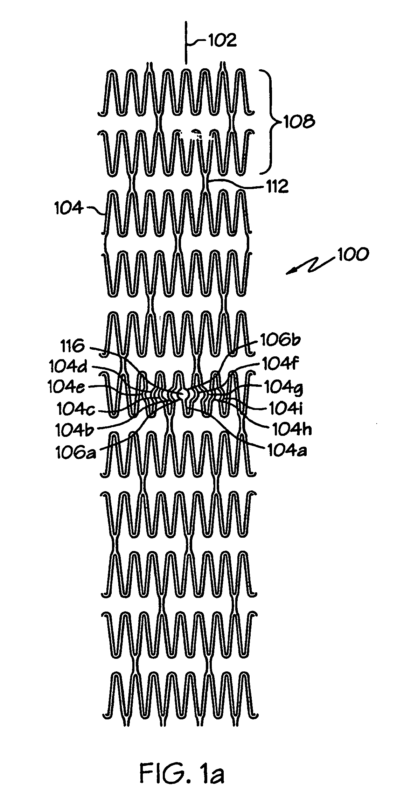

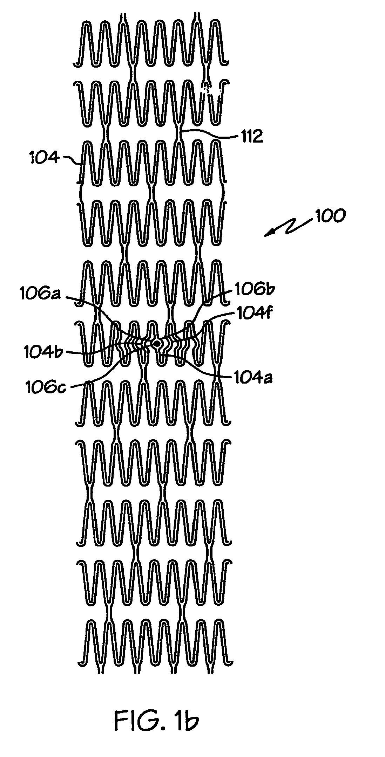

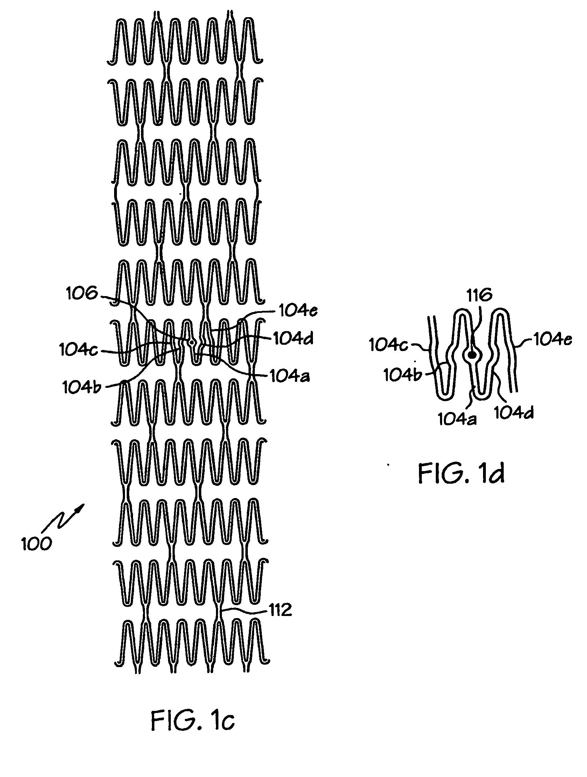

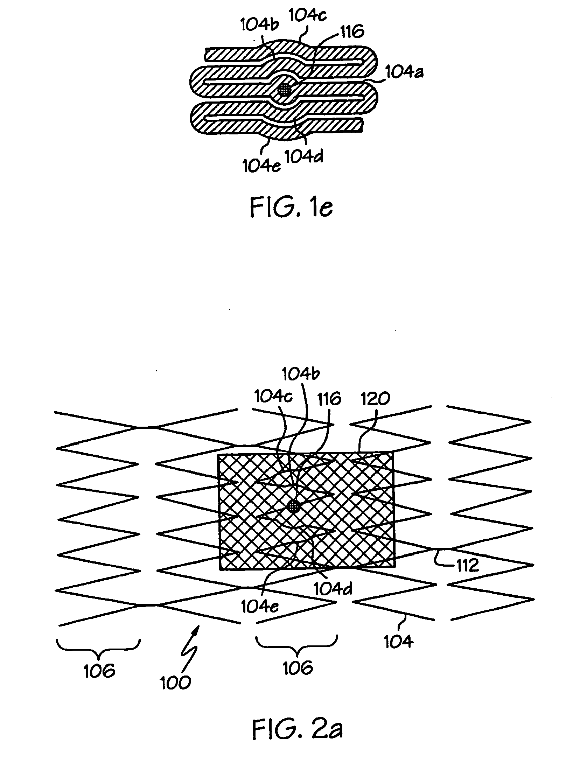

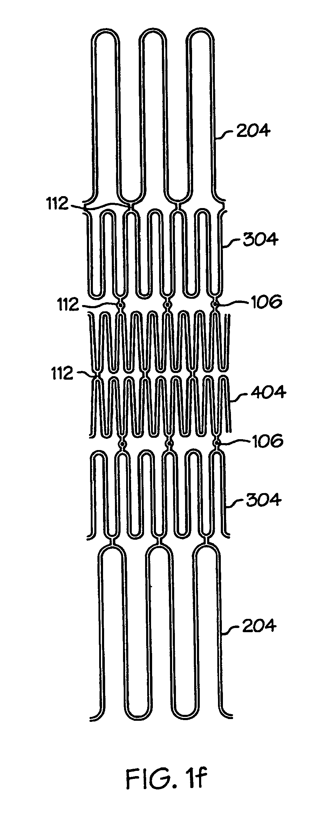

In one embodiment, the invention Is directed to a medical device such as a stent, shown by way of example at 100 in Fig. 1a, comprising a plurality of interconnected struts 104. Struts 104 are arranged in the form of serpentine bands 108. Adjacent serpentine bands are connected to one another via one or more connecting struts 112, as shown in Fig. 1 or directly. In stents where connecting struts are used, the connecting struts may be straight or have one or more bends. It may be curved in a single region or curved over the entirety thereof. The connecting struts may be generally parallel to the longitudinal axis 102 of the stent as shown in Fig. 1 a or may be non-parallel to the longitudinal axis. The ends of a connecting struts may be circumferentially aligned with one another as shown in Fig. 1a or may be circumferentially offset from one another. In the case where the serpentine bands are directly connected, portions of adjacent bands will abut one another.

Stent 100, as shown in Fig. 1a, includes at least one special strut 104a having a first side 106a with a first region of first curvature relative to the longitudinal axis and a second side 106b with a second region of second curvature relative to the longitudinal axis. First region 106a is opposite second region 106b. The first region curves in a direction opposite to the second region relative to the longitudinal axis 102 of the stent. Two or more struts 104b-e adjacent first side 106a of special strut 104a have a shape which is generally complementary to the shape of the first side and two or more struts 104f-i adjacent the second side 106b of the special strut having a shape which is generally complementary to the shape of the second side.

The special strut is more radiopaque in an area bounded by the first and second regions than in the remainder of the strut. Radiopaque area 116 allows for easy location of the stent via fluoroscopy. As shown in Fig. 1a. the radiopaque area is circular. The radiopaque region may also be provided in other shapes, for example in the form of an oval or any other suitable shape. The additional radiopacity may result from a coating or other treatment of the radiopaque area or may simply result from the larger surface area of the strut in the radiopaque region. Using standard fluoroscopic techniques, stainless steel struts having widths of less than 0.0127cms (0.005 inch) are not easily visualized. If the radiopaque region present a width in excess of 0.0127cms (0.005 inch) and desirably significantly in excess of 0.0127cms (0.005 inch) it will be visible under fluoroscopy where the remainder of the strut is not visible.

In embodiment of the invention shown in Fig. 1a, the radiopaque area 116 is sufficiently large that a plurality of struts 104b-i on either side of the special strut have shapes which are curved to complement one another and special strut 104a. The number of complementary struts that are necessary will depend on the size of the radiopaque region as well as on the width of the struts. As the size of the radiopaque region is increased and the width of the struts is decreased, more adjacent struts having complementary shapes will be required. As the size of the radiopaque region is decreased and the width of the struts is decreased, fewer adjacent struts having complementary shapes will be required. In the embodiment of Fig. 1a, the curvature of the struts adjacent the special strut decreases as the distance from the special strut increases.

In the embodiment of Fig. 1a, special strut 104a is located between the ends of the stent. Desirably, the special strut is located at the middle of the stent or between the middle of the stent and one of the ends of the stent. More desirably, the special strut is located between the middle of the stent and a position one third of the way along the stent.

In another embodiment of the invention, as shown in Fig. 1b, the radiopaque area 116 is provided in the form of an insert or rivet, desirable circular in shape, which is inserted in special strut 104a.

In the embodiment of Fig. 1c, fewer struts 104b-e having shapes which complement special strut 104a are provided. Special strut 104a and the struts adjacent thereto are shown in greater detail in Fig. 1 d.

The stents, shown by way of example in Figs. 1a-e, may be modified in a variety of ways. The individual serpentine bands may have fewer or more peaks and troughs and/or may have different curvatures.

The serpentine bands of the stent may be similar to those shown in Fig. 1a-d or may have a different geometry. As shown in Fig. 1e, adjacent struts may be substantially parallel to one another and to the longitudinal axis of the stent.

The length of some of the struts in the serpentine band may differ from the length of other of the struts in the serpentine band, as shown by way of example in Fig.1f in which the special stent and adjacent struts of the invention are not shown. In the stent of Fig. 1f, first serpentine bands 204, located at both ends of the stent, are connected to second serpentine bands 304 which in turn are connected to third serpentine bands 404. First serpentine bands 204 are longer than second serpentine bands 304 which are longer than third serpentine bands 404. Optionally, as shown In Fig. 1f, the number of peaks and troughs may differ between some of the bands. First serpentine bands 204 have fewer peaks and troughs than second serpentine bands 304 which in turn have fewer peaks and troughs than third serpentine bands 404. The stent of Fig. 1f has a 3-6-9-9-6-3 pattern. The number of peaks on adjacent serpentine bands changes from 3 to 6 to 9 to 9 to 6 to 3. The stent may also be provided in an 3-6-9 pattern so that the number of peaks increases along the length of the stent. Patterns with other multiples of a 1-2-3-3-2-1 ratio of peaks are also within the scope of the invention. Bands with different numbers of peaks may be of the same total circumferential length or of different total circumferential length.

The stent of Fig. 1f has special struts in the form of connectors 112 which have an region of enhanced radiopacity 106, in addition to the special strut and adjacent complementary struts as part of one or more serpentine bands similar to those shown in Figs. 1 a-e.

For any of the embodiments disclosed herein, the peaks within a serpentine band may be aligned longitudinally with one another, as shown in the figures, or may extend to different locations along the length of the stent. Similarly, troughs within a serpentine band may be aligned longitudinally with one another or may extend to different locations along the length of the stent.

Stents with helical designs may also be provided with the special struts disclosed herein. By way of example only, the stent of Fig. 1a may be modified so that the serpentine structure is in the form of a helical band.

More generally, any suitable stent design may be modified by providing a special strut as disclosed herein as well as two or more struts circumferentially adjacent to the special strut having shapes which are complementary to the special strut. Two or more struts adjacent a first side of the special strut have a shape which is complementary to the shape of the first side of the special strut and two or more struts adjacent the second side of the special strut have shape which is complementary to the shape of the second side. The first and second sides of the special strut have regions of generally opposing curvature.

Any of the stents disclosed herein may be modified so that different serpentine bands are of different width and/or thickness. The various serpentine bands of the stent may be of the same total circumferential length or of different total circumferential lengths.

The radiopaque region(s), where present, may be provided by any suitable means. The radiopaque portion may be made of gold, palladium, rhodium, platinum, platinum-tungsten, platinum-irldium, iridium, tantalum, silver, molybdenum, iodine and its salts or compounds, barium and its salts or compounds, bismuth and its salts or compounds, tungsten, rhenium, osmium, noble metals and palladium or alloys thereof.

The radiopacity may be provided by plating the radiopaque material onto the stent, by painting it onto the stent, by pressing radiopaque particles into the stent, by swaging a radiopaque material into the stent, by welding or adhesively bonding a radiopaque material onto the stent or via any other suitable means known in the art.

The stent may have one special struts or a plurality of special struts. In the one embodiment of the invention, two special struts are provided. The invention requires that the special strut be provided in the middle of the stent. In the case where the stent has a single special strut, a typical location for the strut the middle of the stent. The struts adjacent the special struts have curvatures which complement the sides of the special struts. Struts on either side of the special strut have opposing curvatures.

The use of special struts such as those disclosed herein is not limited to stents. Special struts such as those shown in Figs. 1a-f, with rediopaque regions and with or without covers, may also be used in other medical devices which comprise struts including filters and occlusive devices.

The invention is also directed to a stent having a longitudinal axis 102, shown by way of example in Figs. 1a-e, comprising a plurality of interconnected struts 104 including at least one strut 104a having a region 116 with an enlarged width. A plurality of first struts 104b, 104c, 104d and 104e are adjacent a first side of strut 104a, and a plurality of second struts 104f, 104g, 104h and 104i are adjacent a second side of strut 104a. The shapes of the first 104b-e and second struts 104f-i taken together complement the shape of the strut 104a having the region 116 with the enlarged width.

The invention is also directed to a stent, such as that shown at 100 in Fig. 1a, in an unexpanded state comprising a plurality of interconnected struts 104. The struts including a central strut 104a, a first strut 104b on one side of the central strut and a second strut on the other side 104f of the central strut. A portion of one side of the central strut is nested within the first strut and a portion of the other side of the central strut nested within the second strut. Desirably, the nested portions of the central strut will be of greater radiopacity as compared with the other struts.

Struts with complementary shapes and radiopaque markers such as those shown in Figs. 1a-e may be used in any suitable stent design. By way of non-limiting examples, they may be used in stents of uniform diameter as well as stents of non-uniform diameter including tapered stents. The struts may be used in stents having uniform or non-uniform rigidity. The struts may be used in stents having uniform wall thickness or non-uniform wall thickness.

Struts with complementary shapes and radiopaque markers such as those shown in Figs. 1a-e Bifurcated stents made in accordance with the invention may also include one or more stent covers.

The use of stent covers is discussed in this disclosure.The use of complementary struts disposed about the enlarged marker region of the stent may allow for improved crimpability of the stent. Stents with such configurations may typically be crimped to smaller diameters, without damaging the struts in the region of the marker, than stents without such configurations.

The inventive stents may also include at least one cover which covers at least a portion of the stent. In the embodiment of Fig. 2a, a schematic Illustration of an inventive stent with a special strut 104a having a radiopaque region 116 and a cover 120 is shown. Cover 120 extends over only a portion of the stent. Stent 100 may be of particular use in the treatment of aneurysms. The stent may be aligned in a vessel having an aneurysm such that covered portion 120 extends over the aneurysm to prevent or minimize blood flow into the aneurysm. Radiopaque region 116 may be used as an aid in positioning the stent such that the covering extends over the region of the aneurysm. Specifically, the radiopaque region may be used, in conjunction with bi-plane fluoroscopy, to denote an axial and polar position on a stent to allow for accurate positioning of the stent to prevent, for example, sidebranch occlusion or incomplete covering of an aneurysm neck.

The invention also contemplates the use of stent covers which extend all the way about the circumference of the stent, stent covers which extend the entire length of the stent and stent covers which both extend all the way about the circumference of the stent and which extend the entire length of the stent. Stents having more than one cover are also within the scope of the invention. Different regions of the stent may be covered by separate covers.

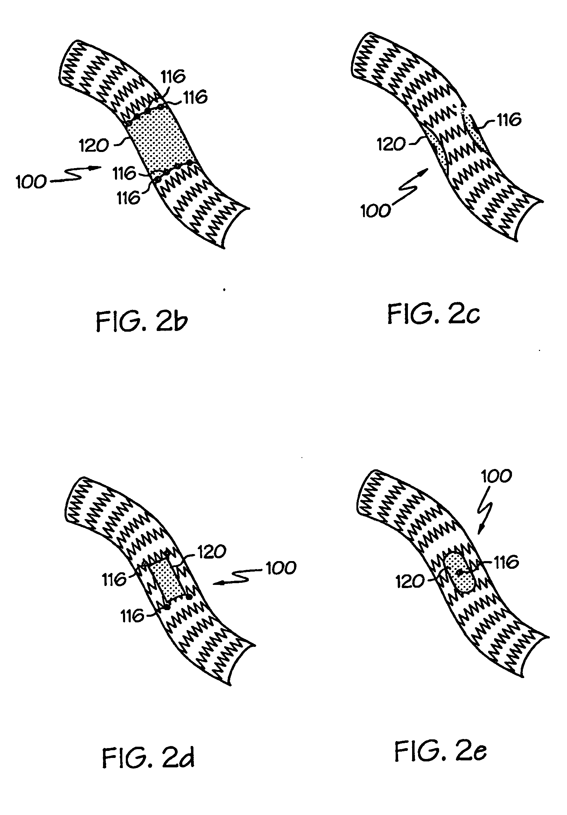

Examples of inventive stents with stent covers are shown in Figs. 2b-2e. Stent 100 of Fig. 2b Includes a cover 120 which extends about the entire circumference of the stent. Stent cover 120 covers only a portion of the stent being shorter in length than the stent. The cover is disposed about the stent towards the middle of the stent. In other embodiments of the invention, the cover may be positioned at either end of the stent. Desirably, at least one and optionally a plurality of radiopaque regions 116 are provided to facilitate locating the cover via fluoroscopy. In the stent of Fig. 2b, a plurality of radiopaque regions 116 are provided about the circumference of the stent at both ends of the stent cover. The radiopaque regions may also be provided at one end of the cover and/or at the middle of the cover.

In the embodiment of Fig. 2c, stent cover 120 extends most, but not all of the way about the circumference of stent 100. The edges of the cover are optionally curved. Radiopaque regions 116 are provided at the middle of cover.1 20 to facilitate visualizing the location of the stent cover under fluoroscopy.

In the embodiment of Fig. 2d, stent 100 includes a stent cover 120, optionally rectangular in shape. Stent cover 120 covers a portion of the stent in the middle. Four radiopaque regions 116 are provided at the corners of the cover to facilitate visualizing the location of the stent cover under fluoroscopy. Additional or fewer radiopaque regions may also be provided in accordance with the invention.

In the embodiment of Fig. 2e, cover 120 is provided in the middle of the stent. Cover 120 covers only a small portion of the stent and does not extend entirely about the circumference of the stent. The cover is substantially oval-shaped and has curved edges. Desirably, at least one radiopaque region 116 is provided. As shown in Fig. 2e, radiopaque region 116 is in the middle of the cover. The radiopaque regions may also be provided along the edges of the stent cover. Additional radiopaque regions may also be provided in accordance with the invention.

The stent cover may be made of any suitable material including polymeric materials and metals. Examples of polymeric materials include polytetrafluoroethylene (ePTFE), unsintered ePTFE. polytetrafluoroethylene, silicone or other polymeric materials including elastomeric materials and any of the graft material disclosed below. Where the cover is made of metal, the metal is desirably in the form of a braid or a foil. An example of a suitable metal is nitinol whether in the form of a braid or a foil or in other suitable forms.

The invention is also directed to stents having covers which cover only selected portions of the stent. Examples of such stents are shown in Figs. 2b-2e. In the embodiments of Figs. 2b-2e, the stent covers are located generally in the middle of the stent. In other embodiments of the invention, stents may be provided with covers such as those shown in Figs. 2b-2e in regions other than the middle. For example, one or both ends of the stent may have a covering in the form of a band as is shown in Fig. 2b or in the form of partial bands as is shown in Figs. 2c-2e. The coverings may also be disposed at a location slightly inward from the end of the stent, for example, one or two strut lengths into the stent. The inventive stents may also have more than one cover.

The inventive stents may be manufactured using known stent manufacturing techniques. Suitable methods for manufacturing the inventive stents include laser cutting, chemical etching or stamping of a tube. The inventive stents may also be manufactured by laser cutting, chemically etching, stamping a flat sheet, rolling the sheet and, optionally. welding the sheet. Other suitable manufacturing techniques include electrode discharge machining or molding the stent with the desired design. The stent may also be manufactured by welding individual sections, for example, circumferential bands, together. Any other suitable stent manufacturing process may also be used.

Any suitable stent material may be used in the manufacture of the inventive stents. Examples of such materials include polymeric materials, metals, ceramics and composites. Suitable polymeric materials include thermotropic liquid crystal polymers (LCP's). Where the stent is made of metal, the metal may be stainless steel, cobalt chrome alloys such as elgiloy, tantalum or other plastically deformable metals. Other suitable metals include shape-memory metals such as nickel-titanium alloys generically known as "nitinol", platinum/tungsten alloys and titanium alloys.

The invention also contemplates the use of more than one material in the inventive stents. For example, some of the serpentine bands may be made from one material and others of the serpentine bands may be made of other materials.

The inventive stents may be provided in mechanically expandable form, in self-expanding form or as a hybrid of the two. Mechanically expandable stents, in accordance with the invention, may be expanded using any suitable mechanical device including a balloon.

The inventive stents may also be provided with various bio-compatible coatings to enhance various properties of the stent. For example, the inventive stents may be provided with lubricious coatings. The inventive stents may also be provided with drug-containing coatings which release drugs over time.

The inventive stents may also be provided with a sugar or more generally a carbohydrate and/or a gelatin to maintain the stent on a balloon during delivery of the stent to a desired bodily location. Other suitable compounds for treating the stent include biodegradable polymers and polymers which are dissolvable in bodily fluids. Portions of the interior and/or exterior of the stent may be coated or impregnated with the compound. Mechanical retention devices may also be used to maintain the stent on the balloon during delivery. To that end, the use of other coatings on the inventive stents is also within the scope of the invention.

The coating may comprise one or more non-genetic therapeutic agents, genetic materials and cells and combinations thereof as well as other polymeric coatings.

Non-genetic therapeutic agents include anti-thrombogenic agents such as heparin, heparin derivatives, urokinase, and PPack (dextrophenylalanine proline arginine chloromethylketone); anti-proliferative agents such as enoxaprin, angiopeptin, or monoclonal antibodies capable of blocking smooth muscle cell proliferation, hirudin, and acetylsalicylic acid; anti-inflammatory agents such as dexamethasone, prednisolone, corticosterone, budesonide, estrogen, sulfasalazine, and mesalamine; antineoplastic/antiproliferative/anti-miotic agents such as paclitaxel, 5-fluorouracil, cisplatin, vinblastine, vincristine, epothilones, endostatin, angiostatin and thymidine kinase inhibitors; anesthetic agents such as lidocaine, bupivacaine, and ropivacaine; anti-coagulants such as D-Phe-Pro-Arg chloromethyl keton. an RGD peptide-containing compound, heparin, antithrombin compounds, platelet receptor antagonists, anti-thrombin anticodies, anti-platelet receptor antibodies, aspirin, prostaglandin inhibitors, platelet inhibitors and tick antiplatelet peptides; vascular cell growth promotors such as growth factor inhibitors, growth factor receptor antagonists, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; and agents which interfere with endogenous vascoactive mechanisms.

Genetic materials include anti-sense DNA and RNA, DNA coding for, anti-sense RNA, tRNA or rRNA to replace defective or deficient endogenous molecules, angiogenic factors including growth factors such as acidic and basic fibroblast growth factors, vascular endothelial growth factor, epidermal growth factor, transforming growth factor αand β, platelet-derived endothelial growth factor, platelet-derived growth factor, tumor necrosis factor α, hepatocyte growth factor and insulin like growth factor, cell cycle inhibitors including CD inhibitors, thymidine kinase ("TK") and other agents useful for interfering with cell proliferation the family of bone morphogenic proteins ("BMP"s",BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Desirable BMP"s are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA"s encoding them.

Cells can be of human origin (autologous or allogeneic) or from an animal source (xenogeneic), genetically engineered if desired to deliver proteins of interest at the transplant site. The cells may be provided in a delivery media. The delivery media may be formulated as needed to maintain cell function and viability.

Suitable polymer coating materials include polycarboxylic acids, cellulosic polymers, including cellulose acetate and cellulose nitrate, gelatin, polyvinylpyrrolidone, cross-linked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactone, polyhydroxybutyrate valerate and blends and copolymers thereof, coatings from polymer dispersions such as polyurethane dispersions (for example, BAYHDROL^{®}), fibrin, collagen and derivatives thereof, polysaccharides such as celluloses, starches, dextrans, alginates and derivatives, hyaluronic acid, squalene emulsions. Polyacrylic acid, available as HYDROPLUS^{®} (Boston Scientific Corporation, Natick, Mass.), and described in U.S. Pat. No. 5,091,205, the disclosure of which is hereby incorporated herein by reference, is particularly desirable. Even more desirable is a copolymer of polylactic acid and polycaprolactone.

The special struts disclosed herein, having portions which are more easily viewed under fluoroscopy, may also be used to mark the location of the regions of the stent which have been provided with any of the drugs or other treatment agents disclosed herein. By way of example, if only a portion of the stent has a treatment agent, or if different portions of the stent have different treatment agents or differing amounts of a treatment agent, radiopaque markers could be used to delineate the different regions of the stent so that the different regions of the stent could be deployed in the desired regions of the vessel with precision. To that end, the invention is also directed to a stent comprising at least two regions having different amounts of one or more treatment agents, the regions delineated via the presence of radiopaque markers between the two regions or at the edges of the regions.

The inventive stents may also be used as the framework for a graft. Suitable coverings include nylon, collagen, PTFE and expanded PTFE, polyethylene terephthalate and KEVLAR, or any of the materials disclosed in US 5,824,046 and US 5,755,770. More generally, any known graft material may be used including synthetic polymers such as polyethylene, polypropylene, polyurethane, polyglycolic acid, polyesters, polyamides, their mixtures, blends and copolymers.

The inventive stents may find use in coronary arteries, renal arteries, peripheral arteries including iliac arteries, arteries of the neck and cerebral arteries. The stents of the present invention, however, are not limited to use in the vascular system and may also be advantageously employed in other body structures, including but not limited to arteries, veins, biliary ducts, urethras, fallopian tubes, bronchial tubes, the trachea, the esophagus, the prostate and the bowels.

Suitable stent delivery devices such as those disclosed in US 6,123, 712, US 6,120, 522 and US 5,957, 930 may be used to deliver the inventive stents to the desired bodily location. The choice of delivery device will depend on whether a self- expanding or balloon expandable stent is used. The inventive stents may be delivered in conjunction with one or more stent retaining sleeves. An example of stent retaining sleeves is disclosed in US provisional application 60/238178.

A method of delivering a stent such as those disclosed herein to a desired bodily location is described but does not form a part of the invention. The stent is disposed about the catheter, delivered to a desired bodily location and expanded, in the case of balloon expandable stent or allowed to expand, in the case of a self-expanding stent.

A method which does not form a part of the invention of positioning a stent or other medical device which is only partially covered in a region of a vessel having an aneurysm comprises the steps of providing a stent or other medical device with a marker for an imaging modality, the stent or other medical device having a covering disposed about the radiopaque marker and a portion of the stent or other medical device adjacent thereto, delivering the stent or other medical device to a region of a vessel having an aneurysm, obtaining an image of the stent or other medical device and determining the location of the marker relative to the aneurysm and repositioning the stent or other medical device if necessary so that the covered portion of the stent or other medical device extends across the aneurysm. The marker will be radiopaque and the imaging modality will be fluoroscopy.

The above disclosure is intended to be illustrative and not exhaustive. This description will suggest many variations and alternatives to one of ordinary skill in this art. All these alternatives and variations are intended to be included within the scope of the claims where the term"comprising"means"including, but not limited to".

## Claims

1. A medical device having a longitudinal axis (102), the device comprising a plurality of interconnected struts (104) including:
at least one special strut (104a) having a first side (106a) with a first region of first curvature relative to the longitudinal axis (102) and a second side (106b) with a second region of second curvature relative to the longitudinal axis (102), the first region opposite the second region, the first region curving in a direction opposite to the second region relative to the longitudinal axis (102) of the device, the special strut (104a) having a radiopaque marker (116) between the first and second regions,
**characterized in that**
two or more struts (104b, 104c, 104d, 104e) adjacent the first side (106a) of the special strut (104a) having a shape which is complementary to the shape of the first side (106a), and
two or more struts (104f, 104g, 104h, 104i) adjacent the second side (106b) of the special strut (104a) having a shape which is complementary to the shape of the second side (106b), wherein the at least one special strut (104a) is in a region between the ends of the medical device.

2. The medical device of claim 1 further comprising a cover disposed about the medical device in a region including the special strut.

3. The medical device of claim 2 wherein the cover (120) extends about the circumference of the medical device.

4. The medical device of claim 2 wherein the cover (120) does not cover the entirety of the medical device.

5. The medical device of claim 1 comprising a plurality of said special strut (104a).

6. The medical device of claim 5 wherein a cover (120) extends about the medical device in the region of each special strut.

7. The medical device of claim 1 wherein the special strut (104a) is located anywhere between the middle of the medical device and a position one half of the way from the middle of the medical device to an end of the medical device.

8. The medical device of claim 1 wherein the curvature of the struts (104b,f; 104c,g; 104d,h; 104e,i) adjacent the special strut (104a) decreases as the distance from the special strut increases.

9. The medical device of claim 1 wherein the radiopaque marker (116) is in the form of a radiopaque material plated, coated or painted on the special strut

10. The medical device of claim 1 wherein the radiopaque marker (116) is in the form of a radiopaque material swaged or welded onto the special strut.

11. The medical device of claim 1 in the form of a stent.

## Patentansprüche

1. Medizinische Vorrichtung mit einer Längsachse (102), wobei die Vorrichtung mehrere miteinander verbundene Streben (104) aufweist, umfassend:
mindestens eine Spezialstrebe (104a), die eine erste Seite (106a) mit einem ersten Bereich einer ersten Krümmung in Bezug auf die Längsachse (102) und eine zweite Seite (106b) mit einem zweiten Bereich einer zweiten Krümmung in Bezug auf die Längsachse (102) aufweist, wobei der erste Bereich dem zweiten Bereich gegenüberliegt, wobei der erste Bereich in eine dem zweiten Bereich gegenüberliegende Richtung in Bezug auf die Längsachse (102) der Vorrichtung gekrümmt ist, wobei die Spezialstrebe (104a) eine strahlungsundurchlässige Markierung (116) zwischen dem ersten und dem zweiten Bereich aufweist,
**dadurch gekennzeichnet, dass**
zwei oder mehr Streben (104b, 104c, 104d, 104e), die an die erste Seite (106a) der Spezialstrebe (104a) angrenzen, eine Form aufweisen, die zur Form der ersten Seite (106a) komplementär ist, und
zwei oder mehr Streben (104f, 104g, 104h, 104i), die an die zweite Seite (106b) der Spezialstrebe (104a) angrenzen, eine Form aufweisen, die zur Form der zweiten Seite (106b) komplementär ist, wobei die mindestens eine Spezialstrebe (104a) in einem Bereich zwischen den Enden der medizinischen Vorrichtung angeordnet ist.

2. Medizinische Vorrichtung nach Anspruch 1, ferner umfassend eine Abdeckung, die auf der medizinischen Vorrichtung in einem Bereich, der die Spezialstrebe umfasst, angeordnet ist.

3. Medizinische Vorrichtung nach Anspruch 2, wobei sich die Abdeckung (120) um den Umfang der medizinischen Vorrichtung herum erstreckt.

4. Medizinische Vorrichtung nach Anspruch 2, wobei die Abdeckung (120) nicht die gesamte medizinische Vorrichtung abdeckt.

5. Medizinische Vorrichtung nach Anspruch 1, umfassend mehrere Spezialstreben (104a).

6. Medizinische Vorrichtung nach Anspruch 5, wobei sich eine Abdeckung (120) um die medizinische Vorrichtung im Bereich jeder Spezialstrebe erstreckt.

7. Medizinische Vorrichtung nach Anspruch 1, wobei die Spezialstrebe (104a) irgendwo zwischen der Mitte der medizinischen Vorrichtung und einer Position auf halbem Weg von der Mitte der medizinischen Vorrichtung bis zu einem Ende der medizinischen Vorrichtung angeordnet ist.

8. Medizinische Vorrichtung nach Anspruch 1, wobei die Krümmung der Streben (104b, f; 104c, g; 104d, h; 104e, i), die an die Spezialstrebe (104a) angrenzen, mit zunehmender Entfernung von der Spezialstrebe abnimmt.

9. Medizinische Vorrichtung nach Anspruch 1, wobei die strahlungsundurchlässige Markierung (116) in Form eines strahlungsundurchlässigcn Materials vorhanden ist, das auf die Spezialstrebe aufplattiert, aufgetragen oder aufgemalt ist.

10. Medizinische Vorrichtung nach Anspruch 1, wobei die strahlungsundurchlässige Markierung (116) in Form eines strahlungsundurchlässigen Materials vorhanden ist, das auf die Spezialstrebe aufgepresst oder aufgeschweißt ist.

11. Medizinische Vorrichtung nach Anspruch 1 in Form eines Stents.

## Revendications

1. Dispositif médical présentant un axe longitudinal (102), le dispositif comprenant une pluralité d'entretoises (104) interconnectées comprenant :
au moins une entretoise spéciale (104a) présentant une première face (106a) avec une première région de première courbure par rapport à l'axe longitudinal (102) et une seconde face (106b) avec une seconde région de seconde courbure par rapport à l'axe longitudinal (102), la première région opposée à la seconde région, la première région s'incurvant dans une direction opposée à la seconde région par rapport à l'axe longitudinal (102) du dispositif, l'entretoise spéciale (104a) comprenant un marqueur radio-opaque (116) entre les première et seconde régions,
**caractérisé en ce que**
deux ou plusieurs entretoises (104b, 104c, 104d, 104e) adjacentes à la première face (106a) de l'entretoise spéciale (104a) présentent une forme qui est complémentaire à la forme de la première face (1 06a), et
deux ou plusieurs entretoises (104f, 104g, 104h, 104i) adjacentes à la seconde face (106b) de l'entretoise spéciale (104a) présentent une forme qui est complémentaire à la forme de la seconde face (106b), ladite au moins une entretoise spéciale (104a) étant dans une région entre les extrémités du dispositif médical.

2. Dispositif médical selon la revendication 1, comprenant en outre un recouvrement placé sur le dispositif médical dans une région incluant l'entretoise spéciale.

3. Dispositif médical selon la revendication 2, dans lequel le recouvrement (120) s'étend sur la circonférence du dispositif médical.

4. Dispositif médical selon la revendication 2, dans lequel le recouvrement (120) ne recouvre pas l'intégralité du dispositif médical.

5. Dispositif médical selon la revendication 1, comprenant une pluralité desdites entretoises spéciales (104a).

6. Dispositif médical selon la revendication 5, dans lequel un recouvrement (120) s'étend autour du dispositif médical dans la région de chaque entretoise spéciale.

7. Dispositif médical selon la revendication 1, dans lequel l'entretoise spéciale (104a) est située à un endroit quelconque entre le milieu du dispositif médical et une position à mi-distance depuis le milieu du dispositif médical jusqu'à une extrémité du dispositif médical.

8. Dispositif médical selon la revendication 1, dans lequel la courbure des entretoises (104b,f; 104c,g ; 104d,h; 104c,i) adjacente à l'entretoise spéciale (104a) décroît au fur et à mesure que la distance depuis l'entretoise spéciale augmente.

9. Dispositif médical selon la revendication 1, dans lequel le marqueur radio-opaque (116) a la forme d'un matériau radio-opaque plaqué, enduit ou peint sur l'entretoise spéciale.

10. Dispositif médical selon la revendication 1, dans lequel le marqueur radio-opaque (116) a la forme d'un matériau radio-opaque estampé ou soudé sur l'entretoise spéciale.

11. Dispositif médical selon la revendication 1 ayant la forme d'un stent.
